# EUROPEAN PATENT SPECIFICATION

(11) **EP 1 401 275 B1**
(45) Date of publication and mention of the grant of the patent: **06.09.2006**
(21) Application number: 02782485.3
(22) Date of filing: 19.06.2002
(51) Int. Cl.: A01N 37/00

(54) **ANTIVIRAL COMPOSITION AND TREATMENT METHOD**
ZUSAMMENSETZUNG UND BEHANDLUNGSVERFAHREN GEGEN VIREN
COMPOSITION ANTIVIRALE ET PROCEDE DE TRAITEMENT

(30) Priority: 06.07.2001 US 899432
(43) Date of publication of application: 31.03.2004
(73) Proprietor: International Flora Technologies, Ltd., Gilbert, AZ 85233-2238 (US)
(72) Inventor: BROWN, James, H., Gilbert, AZ 85233-2238 (US); KLEIMAN, Robert, Gilbert, AZ 85233-2238 (US)
(74) Representative: Lösch, Christoph Ludwig Klaus
(86) International application number: PCT/US2002/019673
(87) International publication number: WO 2003/003833

(56) References cited:
- EP-A- 0 913 158
- US-A- 5 071 879
- US-A- 5 194 451
- US-A- 5 196 410
- US-A- 5 869 529
- US-A- 5 952 392

## Description

### FIELD OF THE INVENTION

The present invention relates to a composition that is useful in the systemic treatment of various virus infections. More specifically, the present invention relates to a systemic antiviral treatment using a mixture of components that result from the interesterification of polyunsaturated jojoba oil with saturated jojoba oil. Methods of preventing or treating viral infections, treating skin or membrane inflammation using such compositions are also disclosed.

### BACKGROUND

Viral infections cause considerable discomfort, disease and can ultimately be fatal to the infected individual. Viruses such as herpes simplex viruses (HSV-1 and HSV-2), cytomegalovirus (CMV), Epstein-Barr virus (EBV), varicella zoster virus (VZV), influenza viruses, human lymphotrophic viruses (e.g., HTLV-1) and human immunodeficiency viruses (e.g., HIV-1) result in significant morbidity and mortality. HSV-1 and HSV-2 are associated with inflammation and lesions of the skin and mucosal membranes, including cold sores, fever blisters and genital herpes lesions. VZV causes shingles and EBV is associated with mononucleosis. Influenza viruses cause flu symptoms and can be fatal. HIV causes acquired immunodeficiency that debilitates and kills infected individuals. While these viruses are latent in some cells, for varying periods of time, generally viral replication results in irreversible destruction of the infected cell producing different clinical manifestations of the diseases they cause.

It is well known that long chain saturated alcohols exhibit a variety of physiological activity. For example, it is known that 1-triacontanol stimulates the growth of plants, see, e.g. U.S. Pat. No.s 4,150,970; 4,874,794; 5070,107; 5,071,879; 5,166,219; 5,194,451; 5,534,554, 5,948,822 and 5,952,392. U.S. Pat. No. 4,670,471 discloses the use of triacontanol, in a suitable carrier, as a treatment for inflammatory disorders such as herpes simplex, eczema, shingles, atopic dermatitis, psoriasis, and the like. The disclosed experiments comprised an aerosol and a mixture of triacontanol and palmitic acid, which was indicated to be as effective as pure triacontanol, and concluded that the aerosol carrier destroyed the effect of triacontanol and that a hydrophilic carrier for triacontanol was necessary to achieve the desired anti-inflammatory effect. There is some reason to believe that the 4,670,471 composition was simply saponified beeswax, which would contain triacontanol and palmitic acid, as indicated, but which would also contain, as substantial constituents, hexacosanolic acid and various other hydrocarbons. Results of gas chromatographic-mass spectrum analysis of various compositions believed to have been used in this patent were not definitive, but suggested that at least some of the other hydrocarbons were very complex mixtures, some of which may contain lower alkanes, esters, acids or alcohols. Whether or not these were found to be effective anti-inflammatory compositions is not known. U.S. Pat. No. 4,670,471 suggests that 5% triacontanol in a branch chain ester base had a certain amount of efficacy for the treatment of HSV-1 dorsal cutaneous infection in guinea pigs and concluded that the active ingredient was the long chain alcohol triacontanol.

The use of non-polar saturated straight chain (C₂₁ to C₃₃) hydrocarbon fractions of beeswax in the treatment of inflammatory skin disorders is disclosed in U.S. Pat. No. 4,623,667.

U.S. Pat. No. 3,863,633 discloses a composition for topical treatment of the eye that comprises a lipophilic substance, a hydrophilic swellable polymer, and from 10 to 80% C₁₂ to C₂₂ surface active alcohols, such as 1-hexadecanol, 1-octadecanol, 1-eicosanol, and 1-docosanol, which serve as a stabilizer for the mixture.

Respecting aliphatic alcohols, one would predict, in the continuum of aliphatic alcohols from C₁₀ to C₃₀, that virucidal activity, at a very low level, may appear (if in vitro studies may be used to predict in vivo results) in C₁₀ to C₁₄ alcohols, which would also be irritants, that virucidal activity disappears in the C₁₆ to C₁₈ range and then reappears in the C₂₀ to C₃₂ range; U.S. Pat. No. 4,874,794, U.S. Pat. No. 5,071,879, U.S. Pat. No. 5,166,219, U.S. Pat. No. 5,194,451 and U.S. Pat. No. 5,534,554.

More Specifically, a C₂₂ saturated aliphatic alcohol, n-docosanol suspended in a surfactant, exhibits potent antiviral activity against viruses including herpes simplex virus, HIV-1 and respiratory syncytial virus in vitro and Friend virus in vivo (Katz, D. H., et al., Proc. Natl. Acad. Sci. USA 88:10825-10829, 1991; U.S. Pat. No. 5,534,554). Progressive binding and uptake of n-docosanol by cells may account for its antiviral activity because preincubation of cells with the alcohol produces optimal antiviral activity. During incubation, 70% of the cell-associated n-docosanol is found in cell membranous components and the remainder is associated with soluble cell fractions (Katz, D. H., et al., Proc. Natl. Acad. Sci. USA 88:10825-10829, 1991). Cell membrane incorporation of n-docosanol does not appear to inhibit virus binding to the cell surface. Instead, early viral protein synthesis is inhibited more than 80% and viruses do not localize to nuclei (Marcelletti, J. F. et al., Drugs of the Future 17(19): 879-882, 1992). Although intracellular metabolic conversions of n-docosanol may account for its antiviral activity, the alcohol is not cytotoxic in concentrations up to 300 mM (Katz, D. H. et al., Annals N.Y. Acad. Sciences, 724:472-488, 1994).

Inactivation of viruses has been reported using C₁₄ to C₂₀ unsaturated long chain alcohols having one to four olefinic bonds. The most effective was γ-linolenyl alcohol, a C₁₈ alcohol with three double bonds at positions 6, 9 and 12; whereas a C₁₈ alcohol with one cis double bond and a C₂₀ alcohol with four double bonds were significantly less effective than the n-docosanol composition reported above (Sands et al., Antimicrob. Agents & Chemother. 15:67-73, 1979).

Some compounds that are structurally related to long-chain aliphatic alcohols also have been associated with antiviral activity. For example, U.S. Pat. No. 4,513,008 discloses the virucidal activity of C₂₀ to C₂₄ linear polyunsaturated acids, aldehydes or alcohols having five to seven double bonds. Compounds having a long chain fatty acyl group, containing at least three or four unsaturated bonds, attached to a nucleoside or nucleoside analogue are disclosed as antiviral treatments in U.S. Pat. No. 5,216,142. Related U.S. Pat. No. 5,276,020 discloses antiviral compounds having a C₁₆, C₁₈ or C₂₀ long chain fatty acid group attached to a nucleoside analogue and a method of treating virus infection using these compounds. Compositions containing oleic acid (C₁₈, one double bond) have also been reported as effective for anti-herpes virus agents (PCT patent application WO 9602244A1). Antimicrobial compositions for topical treatment containing a C₁₅ glycerol monoester of lauric acid or a polyhydric alcohol monoester of lauric acid with a mixture of fatty acids (C₁₀ capric and C₈ caprylic acids) are disclosed in U.S. Pat. No. 5,208,257.

A method of preventing or reducing skin irritation by applying a protective agent containing polymers of C₁₂ to C₂₆ fatty acids prior to exposure to an allergenic agent is disclosed in U.S. Pat. No. 4,076,799. The preferred polymers have two to four carboxy or carboxyl salt groups, preferably the triethanolamine salt of dimerized linoleic acid or its saturated derivative. Other anti-inflammatory polymers containing aromatic heterocyclic residues or acyl residues in homopolymers or heteropolymers (e.g., vinyl esters of C₈ to C₁₈ fatty acids; m.w. 2,000 to 1,000,000) and having greater activity than the component monomers have been disclosed in U.S. Pat. No. 3,946,035.

U.S. Pat. No. 4,513,008 discloses a method of inactivating enveloped viruses using C₂₀ to C₂₄ polyunsaturated acids, aldehydes or alcohols having 5-7 double bonds, and references disclosed therein. Antimicrobial Agents and Chemotherapy 15, 67-73 (1979) (antiviral activity of C₁₄ to C₂₀ unsaturated alcohols having 1-4 double bonds), Snipes et al., Antimicrobial Agents and Chemotherapy 11, 98-104 (1977) (C₂₀ tetraenyl alcohol having low activity) and Symp. Pharm. Effects Lipids (AOCS Monograph N.5) 63-74 (1978) (suggesting lower antiviral activity for saturated long-chain alcohols).

A surfactant/erucyl alcohol (cis-13-docosen-1-ol) suspension has been tested and reported for antiviral activity. Surfactant/erucyl alcohol suspensions did not have a direct virucidal effect. That is, incubation of the HSV-2 virus with the surfactant/erucyl alcohol suspension for 2 hours did not inactivate the virus as measured by subsequent plaque formation on Vero cells. The surfactant/erucyl alcohol suspension was toxic to Vero cells when added to cultures at concentrations where n-docosanol is effective (2-15 mM). However concentrations that were tolerable to the cells (≤ 1 mM) showed significant inhibition of HSV-2 plaque production (to 93%). Moreover, no cellular toxicity was observed at 1 mM erucyl alcohol. The effective concentration required to inhibit plaque formation by 50% for erucyl alcohol (EC₅₀ = 0.15 mM) was 60-fold lower than the concentration required for n-docosanol (EC₅₀ = 9 mM). Similarly, the antiviral activity of the trans-isomer of the C₂₂ mono-unsaturated alcohol, brassidyl alcohol (trans-13-docosen-1-ol) has been reported. Brassidyl alcohol exhibits antiviral efficacy similar to n-docosanol. The cellular toxicity of brassidyl alcohol was significantly less than that of erucyl alcohol. Based on these results, it can be seen that the addition of a single cis (but not trans) double bond at position 13 of the C₂₂ aliphatic alcohol greatly increased antiviral activity. The alcohol with the trans double bond was less toxic than the alcohol with the cis double bond.

Erucamide (cis-13-docosenoamide; m.w. = 337.59) is a C₂₂ long-chain amide with a single double bond similar in structure to erucic acid. The C₂₂ amide was toxic to Vero cells when used at 3 mM or greater concentrations, similar to the toxicity seen with erucyl alcohol and n-docosanoic acid. When suspensions of erucamide at 3 mM to 15 mM were added to the cultures, the cells became rounded and detached from the plate. At lower concentrations of erucamide in the suspension, significant antiviral activity was seen. At tolerable concentrations of erucamide (.Itoreq.1.7 mM), the antiviral activity of the erucamide suspension was less than essentially equivalent concentrations of suspensions of erucyl alcohol but greater than that of suspensions of n-docosanol, n-docosane, n-docosanoic acid or brassidyl alcohol. That is, the percent inhibition of plaque formation for erucamide suspensions was 78% at 1.7 mM, 68% at 1.5 mM, 58% at 1.2 mM, 44% at 0.89 mM, 42% at 0.59 mM and 34% at 0.03 mM. Thus, the C₂₂ amide exhibits significant antiviral activity at dilutions tolerable to cells but has increased cytotoxicity relative to the C₂₂ saturated aliphatic alcohol (n-docosanol) and similar to that seen with the corresponding C₂₂ mono-unsaturated erucyl alcohol.

Thus, there is a current and continuing need for improved antiviral medicaments. The present invention is a unique composition comprising a combination of mono-unsaturated fatty alcohols and is effective in the systemic treatment of virus-induced disease and in the prevention or inhibition of infection by disease-causing virus. Other compositions according to the present invention may include salts of long chain fatty acids and/or mixed esters.

### SUMMARY OF INVENTION

The present invention is embodied in methods for preventing, inhibiting and treating virus diseases in humans or other animals, comprising intravenous, intramuscular, transmucosal, transdermal or oral introduction into the human or other animal to be treated of a composition comprising alcohols of the general formula R¹CH₂-OH, wherein R¹ comprises CH₃-(CH₂)₇-CH=CH-CH₂-(CH₂)ₓ-, and x is 6, 8, 10 and 12 in a physiologically compatible carrier, and to compositions suitable for carrying out such methods. Other compositions according to the present invention may include salts of long chain fatty acids and/or mixed esters.

### BRIEF DESCRIPTION OF THE DRAWING

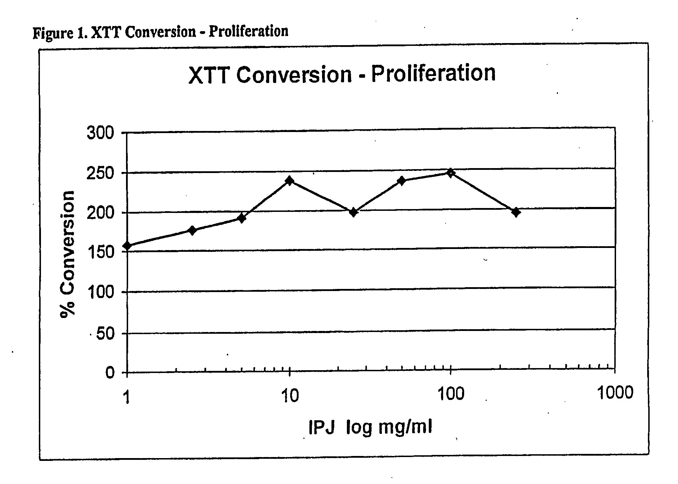

- Figure 1: shows the conversion-proliferation profile of the composition according to the present invention.

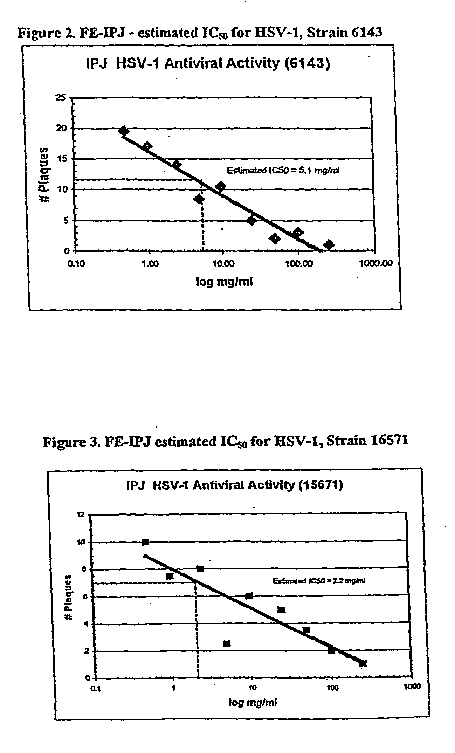

- Figure 2: illustrates the antiviral activity of the composition according to the present invention with respect to HSV-1, strain 6143.
- Figure 3: illustrates the antiviral activity of the composition according to the present invention with respect to HSV-1, strain 16571.

### DETAILED DESCRIPTION OF PREFERRED EMBODIMENTS

The present invention is a composition, and method thereof, that is useful for the treatment of various viral infections. The method may be carried out using compositions comprising alcohols of the general formula R¹CH₂-OH, wherein R¹ comprises CH₃-(CH₂)₇₋CH=CH-CH₂-(CH₂)ₓ-, and x is 6, 8, 10 and 12, or comparable compositions that may also include other physiologically active constituents that do not interfere with the efficacy of the primary composition of the present invention. These other physiologically active constituents may comprise salts of long chain fatty acids and/or mixed esters, as described further below.

In one embodiment, the compositions according to the present invention comprises fatty alcohols and may further comprise mixed chain esters and unreacted wax esters. In this embodiment, compositions according to the present invention may be obtained by the base catalyzed alcoholysis reaction between a wax ester, such as jojoba oil, and an alkyl alcohol, such as isopropyl alcohol. In the alcoholysis reaction, examples of the base catalyst materials include, metal alkoxides and especially alkali metal alkoxides, inorganic hydroxides, especially alkali metal hydroxides, and the like such as NaOCH₃ (sodium methoxide), NaOCH₂CH₃ (sodium ethoxide) and their potassium, calcium or lithium counterparts, KOH and NaOH (e.g., anhydrous alkali metal hydroxides in alcohol solution, with the alcohol of the solution being the alcohol used in the reaction).

The fundamental reactions used in the practice of the present invention may be generally considered in the following manner. Starting materials could include:
Unsaturated wax esters (with jojoba wax esters being the preferred unsaturated wax ester),

   **I.** R¹-COO-CH₂-R¹,

   wherein R¹ comprises CH₃-(CH₂)₇-CH=CH-CH₂-(CH₂)ₓ-, and x is 6, 8, 10 and 12, and
An alcohol,

   **II.** R²-OH,
wherein R² is an alkyl group or other aliphatic group, preferably of 1 to 12 carbon atoms, more preferably an *iso*-alkyl group, and most preferably an isopropyl group,

Typical product components from this first embodiment synthetic reaction used in the practice of the present invention may include:
Mixed chain esters (iso-propyl esters where isopropyl alcohol was used)

   **III.** R¹-COO-R² (R¹-COO-CH-(CH₃)₂) and/or
Fatty mono-unsaturated alcohols comprising:

   **IV.** R¹CH₂-OH

The basic reaction, which may be used in this first embodiment of the preparation of the compositions of the present invention, may include at least the following procedures.

In and **II** + catalyst => **I**r, **III,** and **IV.**

The subscripts n and r respectively represent: the naturally occurring distribution of wax esters (n) and the randomized distribution of wax esters resulting from rearrangements that occur during the reactions (r). It is to be noted that mixing of the reaction products will give compositions with a wide range of melting points.

A process for producing the composition according to the present invention may comprise the steps of:
a) providing a composition comprising at least one wax ester, such as jojoba oil,
b) adding an alcohol, e.g., having from 1 to 12 carbon atoms, to said composition,
c) adding a catalyst and sufficient heat to thereby effect alcoholysis on said wax ester mixed with said alcohol, and effect interesterification between wax esters.

In preparing the composition according to the present invention, refined wax ester is introduced into an appropriate vessel capable of excluding air and equipped with devices for stirring, heating and cooling. The starting materials are first dried under vacuum at a temperature of 90 °C to remove most or all moisture. Anhydrous alcohol is then added to the mixture with the amount of alcohol ranging from about 20 % to about 50% of the weight of the wax esters. The reactor is sealed and heat is applied to bring the temperature of the reaction mixture to about 70-75 °C. It is important that air is excluded and that the reactor vented through a condenser to recover any unreacted alcohol. When the temperature has reached 70-75 °C, a first addition of catalyst (e.g., a catalyst for alcoholysis and interesterification such as sodium methoxide) is made. The amount added ranges from about 0.05 or from 0.1 % to about 0.6 % by weight of the wax esters with about 0.3 % being preferred. After about 2 hours, a sample is taken and analyzed for the presence of unreacted wax esters. If the unreacted wax ester content is greater than about 35% by weight, and it is desired to have a higher level of mono-unsaturated fatty alcohols present in the reaction mixture, a second addition of catalyst is made, about 0.1% by weight of the original amount of wax ester. The reaction is then continued for an additional one hour. The reaction mixture is then sampled and analyzed again. If the residual starting material content falls between about 25-35%, then the reaction may be considered to be complete. Heating is discontinued but no cooling is applied. If the reaction is considered incomplete, a third catalyst addition may be made and the reaction continued as previously described. Any remaining catalyst can be neutralized and deactivated by the addition of citric acid. After about 15 minutes of agitation the neutralized catalyst (sodium citrate) is removed by filtration. Once the catalyst has been removed, any remaining alcohol can be distilled from the product and the recovered alcohol should be kept absolutely dry in order to be able to be used again.

When using jojoba oil as the source of the wax esters, the composition of the naturally occurring fatty substituent in jojoba oil are 1%, 44%, 45%, and 9% for C₁₈, C₂₀, C₂₂, and C₂₄, respectively. The alcoholysis reaction described above would provide the long chain monounsaturated alcohols in equivalent proportions. Thus, the most preferred embodiment of the composition according to the present invention comprises long chain monounsaturated fatty alcohols wherein said alcohols are comprised of relative proportions of : octadecenol is 1%, eicosenol is 44%, docosenol is 45%, and tetracosenol is 9%.

In another embodiment, wax esters may be hydrolyzed to produce monounsaturated long chain alcohols and salts of long chain fatty acids. This reaction proceeds as described below:

The fundamental reactions used in the practice of the present invention may be generally considered in the following manner. Starting materials could include:
Unsaturated wax esters (with jojoba wax esters being the preferred unsaturated wax ester),

   **I.** R¹-COO-CH₂-R¹,

   wherein R¹ comprises CH₃-(CH₂)₇-CH=CH-CH₂-(CH₂)ₓ-,and x is 6,8,10 and 12, and
An alkali metal hydroxide

   **II.** M-OH,
wherein M⁺ is a metal ion, preferably the alkali metals potassium or sodium.

Typical product components from this second embodiment synthetic reaction used in the practice of the present invention may include:
Salts of long chain fatty acids

   **III.** R¹-COO⁻M⁺

   and
Mono-unsaturated fatty alcohols comprising:

   **IV.** R¹CH₂-OH

The basic reaction, which may be used in this second embodiment of the preparation of the compositions of the present invention, may include at least the following procedures.

**I** and **II** => **III**, and **IV**.

Preferably, compositions of matter comprising waxes, oils and/or fats (lipids) and contain ester and are subjected to an alkaline hydrolysis reaction to produce a non-foaming, substantive composition with unique surfactant properties that may be used as an active ingredient, as described herein, or also a carrier for application of other active ingredients, e.g., as a carrier base for application of cosmetic, pharmaceutical or other active ingredients. Commercially available bio-based extracts that have high unsaponifiables include, but are not limited to, candelilla wax, carnuba wax, jojoba oil, and lanolin.

The extracts used as starting materials for the hydrolysis reaction according to the method of the present invention may also be alkoxylated, polymerized, acetylated, oxidized, reduced, concentrated, partial hydrogenated, interesterified, double bond modified, randomized, refined, or otherwise modified before the hydrolysis reaction.

The products from the hydrolysis reaction of organic materials that produce unsaponifiables comprises a mixture of: a) polar hydrophilic salts (saponifiables); and b) non-polar, lipophilic materials (unsaponifiables), which comprise at least mono-unsaturated fatty alcohols and salts of long chain fatty acids according to formulas III and IV above, with the possibility of other materials also present, depending on the source, state and form of the initial reactant.

The composition of materials created by this method are produced by the reaction of aqueous alkali metal hydroxides, e.g., NaOH, LiOH, KOH (the preferred hydroxide), Ca(OH)₂, Mg(OH)₂, and the like, with organic lipid compositions, usually plant extracts, oils, fats, or waxes (of the extracts or derivatives of the extracts), and preferably as long chain esters.

Jojoba oil may be examined as an example case. Jojoba oil contains various proportions of long chain diunsaturated esters. Hydrolysates of refined jojoba oil are nearly a 55:45 mixture of polar hydrophilic long chain salts (alkali salts) and relatively non-polar lipophilic materials (fatty alcohols). The lipophilic fraction is the unsaponifiable materials according to the definition used in this document. The carbon chain lengths of both of these jojoba hydrolysates include and vary from C₁₈ to C₂₄ and have ω-9 double bonds as part of each molecule. It has been found that the combination of saponifiable and unsaponifiable fractions of the hydrolysates according to the present invention has properties that aid in the formulation of cosmetic, pharmaceutical, and other compositions.

The products that result from the hydrolysis of the lipids containing high percentages of unsaponifiable materials, as created during the practice of the present invention, whether used neat, blended, dissolved, dispersed, or emulsified with excipients, solvents, or carriers, can contain and impart useful properties to applied surfaces. These surfaces may be animate surfaces, particularly human skin, plant surfaces, and even the surfaces of inanimate objects, for example objects of wood, fiber, or plastic. The properties can include, but are not limited to, substantivity, emulsification, hydration, and the like.

In manufacture, a measured quantity of potassium hydroxide pellets are added into the steam kettle with a measured quantity of distilled, deionized, or reverse osmosis purified water. The amount of potassium hydroxide employed in order to completely saponify the free organic acid and/or organic acid ester can accordingly be calculated from the Saponification Value of the starting material and will, in theory, be the stoichiometric amount. In practice, however, it is preferred to employ slightly less than the stoichiometric amount of potassium hydroxide in order to ensure that the Hydrolysates that are formed are not contaminated with unused alkali. The amount of potassium hydroxide employed can be considerably less than the stoichiometric amount, for example, as little as 50% of the stoichiometric amount or less may be used depending upon the desired result. It is to be understood, however, that an amount of potassium hydroxide in excess of the stoichiometric amount, for example, up to 10% more than the stoichiometric amount, can be employed if complete saponification of the organic acid or ester is to be achieved. Excess potassium hydroxide remaining at the end of the reaction may be removed by traditional methods.

The potassium hydroxide pellets and water are stirred together with the propeller mixer until the potassium hydroxide pellets are dissolved. It is important to note; for safety purposes, that heat is generated during this step and the mixture is quite caustic. Individuals nearby should wear gloves, eye and face protection, and clothing protection to avoid burns, both thermal and chemical.

Next, a measured quantity of a refined or derivatized organic material containing a high proportion of unsaponifiables, such as jojoba oil, is gently added to the steam kettle, taking care not to splash the caustic solution contained therein.

The steam kettle is heated to 90-95°C and held at that temperature range under constant agitation for two hours. At this point, the resultant mixture should be pH tested. If the solution pH is greater than 10.0, continue heating the mixture under constant agitation at 90-95°C. Retest the solution periodically until the pH is 10.0 or less.

Once the pH is 10.0, or less, withdraw a sample for analysis. This sample should be analyzed by such methods as chromatography or by another like or similar method, to show that the reaction has proceeded as desired.

The resultant hydrolysate may then be diluted by adding a second measure quantity of water, or other diluent, to the steam kettle and stirred with the mixing propeller. Heat should be continuously applied, less than 80°C, until the mixture is homogeneous.

Once homogeneous, the hydrolysate mixture is cooled to 60°C while continuing the mixing with the propeller. The hydrolysate mixture may then be transferred to a holding container and allowed to cool to room temperature before sealing the holding container.

A carrier may be provided to assist in transfer of the composition according the present invention to the animal, including humans, being treated. The composition of the carrier is not critical so long as the carrier is physiologically compatible with the blood and tissues of the human or other animal to be treated and is substantially free from any interfering physiological effect.

Optimally the compositions effectively reduce the viral titre overall in the treated individual, particularly for systemic treatment, and in lesions, particularly for topical treatment of affected areas of the skin or mucous membrane. The disclosed methods of treatment also reduce symptoms of viral infection (e.g., pain associated with viral-caused lesions) and promote more rapid healing than seen without treatment.

The method of the present invention includes administration of a composition containing the active ingredient and a surfactant to a human or animal to treat or prevent viral infection. Administration is preferably to the skin or a mucous membrane using a stick, such as lipstick or lip balm), cream, lotion, gel, ointment, suspension, aerosol spray or semi-solid formulation (e.g., a suppository), all formulated using methods well known in the art. Applications of the compositions containing the active ingredient and surfactant effective in preventing or treating a viral infection consist of one to ten applications of 10 mg to 10 g per application for one to fourteen days. Applications are generally once every twelve hours and up to once every four hours. Preferably two to four applications of the composition per day, of about 0.1 g to 5 g per application, for one to seven days are sufficient to prevent or treat a viral infection. For topical applications, the compositions are preferably applied to lesions daily as soon as symptoms (e.g., pain, swelling or inflammation) are detected.

The compositions and methods are useful for preventing or treating a variety of viral infections such as those caused by herpes viruses including, but not limited to, HSV-1, HSV-2 and HSV-6, cytomegalovirus (CMV), Epstein-Barr virus (EBV) and varicella zoster virus (VZV), by influenza viruses, human lymphotrophic viruses (e.g., HTLV-1), human immunodeficiency viruses (e.g., HIV-1), papilloma virus and respiratory syncytial virus.

Compositions suitable for intravenous or intramuscular injection into human or animal patients comprise alcohols of the general formula R¹CH₂-OH, wherein R¹ comprises CH₃₋(CH₂)₇-CH=CH-CH₂-(CH₂)ₓ-, and x is 6, 8, 10 and 12, in a suitable carrier for intravenous or intramuscular injection. For example, a suspension of from 0.1 mg/ml to 300 mg/ml of the composition according to the present invention suspended in a carrier solution of isotonic sodium chloride solution containing a suitable preservative, such as 0.1 to 1.5% benzyl alcohol, stabilizers such as from 0.25 to 1% carboxymethylcellulose sodium and 0.005 to 0.1% polysorbate 80, and sufficient sodium hydroxide or hydrochloric acid to adjust the pH to 5.0 to 7.5, all percentages by weight, may be used either intravenous or intramuscular injection.

Another composition suitable for intravenous or intramuscular injection into the human or animal patient may the composition according to the present invention suspended in a carrier solution of ethyl alcohol (1-10%), glycerin (10-20%) and water (balance 70-89%), along with suitable preservatives.

Such compositions may be injected in suitable amounts to provide a dose to the patient of from 0.1 mg/50 kg body weight to 2 gm/50 kg body weight. It is desirable to achieve and maintain a level of the specified composition in the body in the range of at least about 0.1 mg/kg of body weight.

The composition to which the present invention is directed may effectively be introduced through the mucus membrane system of the human or animal patient. Such introduction may be, for example, through the vaginal, anal, oral or nasal membranes. The above liquid compositions, in a suitable liquid carrier may, for example, be used for trans-mucus membranal introduction of such composition into the circulatory system of the human or animal to be treated by, for example, introducing such liquid as an aerosol into the oral or nasal passages or as a liquid into the vaginal or anal passage of the body where these compounds inactivate virus locally, inhibit the passage of virus into the membrane, and pass through the membrane into the circulatory system of the patient where the compounds act as inhibitors of viral activity and infectivity and inactivate virus. In the latter applications, however, gels, creams or suppositories are more conveniently used.

Likewise, the alcohol-containing composition may be introduced through the anus where it also inactivates virus, inhibits the passage of virus into the membrane, and passes through the membrane into the circulatory system of the patient where it acts as an inhibitor of viral activity and infectivity and inactivates virus in the circulatory system and cells nourished by the circulatory system. The specified composition may be in any physiological acceptable form such as in cream or suppository compositions. An exemplary suppository may consist essentially of the composition according to the present invention alone or in a concentration of from 0.05 mg/gm of carrier to 400 (or higher) mg/gm of carrier. Cocoa butter is a commonly used suppository carrier component, alone or in mixture with, for example, tartaric acid and malic acid. Polyethylene glycols of suitable molecular weight are also suitable suppository carriers. Suppositories may also include a preservative such as methylparaben or benzethonium chloride, and such acid or base components as are desired to adjust the pH to the range of about pH 5 to pH 7.5. Any of the above, or other, suitable suppository carrier compositions may be used with composition to form a suitable contraceptive and/or anti-viral suppository. The suppository, to be commercially and aesthetically acceptable, must be a solid at ambient room temperature, i.e. generally in the range of about 27 °C., and must melt at or slightly below normal body temperature, i.e. in the general range of about 37 °C. These temperatures are, of course, only general ranges, and the precise melting point is not critical.

Trans-membranal introduction of the composition according to the present invention may be accomplished by introducing small amounts of such alcohols neat, but such introduction is difficult to control and not efficient.

Cream and gel compositions in concentrations of from about 0.1 mg/ml to 300 mg/ml (or higher) in a suitable cream or gel carrier may also be used effectively. Such a gel may, for example, comprise a suspension agent such as Carbomer® polyacrylic acid cross-linked with allyl sucrose, polyethylene glycol, water and suitable preservatives. A suitable cream base may, for example, comprise white petrolatum, polyoxyethylene stearate, cetyl alcohol, stearyl alcohol, propylene glycol, isopropyl myristate, sorbitan monooleate and water along with suitable preservatives adjusted to a pH of from pH 5 to pH 7.5.

The composition of interest here may also be introduced for trans-membranal passage into the human or animal patient's circulatory system, as well as a prophylaxis against infection from airborne virus, through inhalation of the composition according to the present invention in a suitable physiologically acceptable carrier. The liquid compositions mentioned before may, for example, be packaged in a nebulizer and introduced through nasal or oral passages in the customary manner. An exemplary composition comprises the composition according to the present invention suspended in aerosol propellant such as trichloromonofluoromethane and/or dichlorodifluoromethane, along with diluents, preservatives, pH adjusting reagents, etc. The exemplary aerosol composition delivers essentially pure composition to the mucus membrane. An exemplary ear drop composition delivers essentially pure composition to the tympanic membrane. Comparable liquid drops may be applied using appropriate droppers to the eyes, ears and mouth for application to and passage through the membranes in these respective organs.

All trans-membranal compositions may, in addition to other ingredients, may also include penetration enhancers. A number of such enhancers are known as penetration enhancers and may be used in the compositions of this invention. One such vehicle is dimethyl sulfoxide, which is described in U.S. Pat. No. 3,551,554. Other such penetration enhancers are disclosed in U.S. Pat. Nos. 3,989,816; 3,991,203; 4,112,170; 4,316,893; 4,415,563; 4,423,040; 4,424,210; 4,444,762, sometimes referred to as Azone® .

The anti-viral effectiveness of these alcohols has been established in in-vitro tests, as demonstrated in the following example.

In order to assess the antiviral activity of the composition according to the present invention, a standard plaque assay was used. The plaque-reduction assay is commonly used in the pharmaceutical industry to screen material for antivual activity. In this assay mammalian cells susceptible to viral infection are grown in tissue culture. These cultures were exposed to nontoxic concentrations of the composition according to the present invention and subsequently to live virus. The number of cells that become infected, die and spread virus to surrounding cells are measured by counting those areas void of viable cells (i.e., plaques) in the culture. Therefore, the first step in conducting the assay is to determine the cytotoxicity of the tested composition in Vero cells. Vero cells are epithelial-like cells originally derived from the kidney of the normal African green monkey. These mammalian cells are susceptible to viral infection. Cytotoxicity of the composition according to the present invention was determined using a microculture tetrazolium assay (MTA).

Nontoxic concentrations of the compositions were then used to determine, via plaque-reduction assay, the viral efficacy (i.e., antiviral properties) of the compositions when used to treat Vero cells exposed to Herpes Simplex Virus Type 1 (HSV-1).

The compositions were suspended in Eagles Minimum Essentials Medium containing 10 mg/ml of PLURONIC F-68® (supplied by BASF), a nonionic surfactant that had been heated to 50 °C. The purpose of the surfactant was to facilitate dispersion of the compositions, which.are not readily water soluble, into a water-based medium. The compositions were added to different aliquots to concentrations of 25%, 10%, 5%, 2.5%, 1%, 0.5%, 0.25%, and 0.1%.

Confluent Vero cells in 96-well microculture plates were treated with suspensions prepared as above for approximately 72 hours in a humidified incubator (36 - 38 °C in 5 7 % CO2). The suspensions were aspirated and the cultures gently washed with sterile phosphate buffered saline. Cellular viability was quantified by performing a microculture tetrazolium assay (MTA) using 2,3-bis(2-methoxy-4-nitro-5-sulfophenyl)-5[(phenylamino)carbonyl]-2H-tetrazolium hydroxide (abbreviated as "XTT"). A 1-mg/ml solution of XTT supplemented with 6.12 µg/ml phenazine methylsulfate was added to each well for 3-5 hours at 36-38 °C in 5-7% CO2.

The XTT reagent penetrates into the cells. In viable cells, the XTT reagent is metabolized by the mitochondria and produces soluble, blue formazine dye. If the cells in the well that have been treated with the suspensions are dead or unable to metabolize the XTT (i.e., not viable), the amount of dye produced will be less than that produced in viable cultures. The amount of dye produced during metabolism of the XTT reagent was quantified from optical density (OD) readings obtained using a spectrophotometer (450 nm). Percent cellular conversion in treated cells was calculated by comparing OD readings of control cells. XTT metabolism is compared to untreated control cultures and expressed as a percentage of the untreated controls. Because the MTA assay is a measure of viable cells, it can also provide information regarding the proliferation (increased cellular division) of treated cells relative to untreated control cells. The results of the MTA assay are shown in Table 1 below.

**Table 1. MTA Assay**

| Test Concentration | Mean OD (n = 3) | % Cellular Proliferation/Metabolism |
|---|---|---|
| 250 | 1.559 | 194.8 |
| 100 | 1.967 | 245.8 |
| 50 | 1.891 | 236.3 |
| 25 | 1.574 | 196.7 |
| 10 | 1.905 | 238.0 |
| 5 | 1.531 | 191.3 |
| 2.5 | 1.408 | 175.9 |
| 1 | 1.257 | 157.0 |
| 0 | 0.800 (n = 6) | n/a |

### DETERMINATION OF ANTIVIRAL ACTIVITY

The virus VML-6143 strain of Type-1 Herpes Simplex Virus (HSV-1), which is sensitive to all known anti-HSV drugs, and strain 15671, which is resistant to acyclovir, penciclovir, and ganciclovir, were used to infect Green monkey kidney cells, (Vero cells, ATTC CCL81). This was done to determine the effects of using suspensions of the present compositions on efficiency of plaque formation. Vero cells were cultured and maintained in Eagles Minimal Essentials Medium (E-MEM) supplemented with 2-10% heat inactivated Fetal Bovine Serum (FBS), 100 units/ml penicillin, 2.5 µg/ml Amphotericin B, and 10 µg/ml Gentamicin, at 36-38 °C in a humidified chamber atmosphere of 5-7% CO₂. Control surfactant suspensions or suspensions containing the composition according to the present invention were added to 90%=100% confluent Vero cultures on the day before infection. After 24 hours, the HSV-1 virus was added to the cultures using approximately 30 plaque forming units (PFU) per well in 12-well plates. Approximately 48 hours after the addition of HSV-1, cultures were washed once with phosphate buffered saline. The cells were fixed with SafeFix, stained with 0.8% Crystal Violet and allowed to air dry. Plaques were counted with the aid of a stereoscope and a hand-held electronic colony counter. The data presented are the averages (means) of the duplicate cultures. The data were graphically analyzed to determine the concentration that produced 50% (IC₅₀) reduction of plaques.

The composition according to the present invention demonstrated dose dependent inhibition of plaque formation, indicating direct antiviral activity against the standard clinical HSV-1 control, strain 6143, and the acyclovir resistant HSV-1, strain 15671. Strain 6143 plaque data are presented in Table 2. Strain 15671 data are present in Table 3.

**Table 2. HSV-1, Strain 6143 - anti-plaque activity of the tested composition**

| Treatment Groups | # Plaques Replicate 1 | # Plaques Replicate 2 | Average Plaque per Well |
|---|---|---|---|
| 250 mg/ml | 1 | | 1 |
| 100 mg/ml | 2 | 4 | 3 |
| 50 mg/ml | 3 | 1 | 2 |
| 25 mg/ml | 6 | 4 | 5 |
| 10 mg/ml | 6 | 15 | 10.5 |
| 5 mg/ml | 3 | 14 | 8.5 |
| 2.5 mg/ml | 15 | 13 | 14 |
| 1 mg/ml | 18 | 16 | 17 |
| 0.5 mg/ml | 16 | 23 | 19.5 |
| Controls | | | |
| Vero Culture cont. (no virus, no suspensions) | 0 | 0 | 0 |
| HSV Virus Cont. (virus, no suspensions) | 20 | 23 | 21.5 |
| Media cont. (no virus, no suspensions) | 0 | 0 | 0 |

**Table 3. HSV-1, Strain 15671 - anti-plaque activity of the tested composition**

| Treatment Groups | # Plaques Replicate 1 | # Plaques Replicate 2 | Average Plaque per Well |
|---|---|---|---|
| 250 mg/ml | 1 | 0 | 1 |
| 100 mg/ml | 3 | 1 | 2 |
| 50 mg/ml | 4 | 3 | 3.5 |
| 25 mg/ml | 6 | 2 | 4 |
| 10 mg/ml | 11 | 1 | 6 |
| 5 mg/ml | 3 | 2 | 2.5 |
| 2.5 mg/ml | 10 | 6 | 8 |
| 1 mg/ml | 6 | 9 | 7.5 |
| 0.5 mg/ml | 9 | 11 | 10 |
| Controls | | | |
| Vero Culture cont. (no virus, no suspensions) | 0 | 0 | 0 |
| HSV Virus Cont. (virus, no suspensions) | 13 | 15 | 14 |
| Media cont. (no virus, no suspensions) | 0 | 0 | 0 |

Estimated concentrations that inhibited the number of plaques by 50% relative to non-treated control wells were 5.1 mg/ml for strain 6143 and 2.2 mg/ml for strain 15671. It is surprisingly noted that the composition according to the present invention is not only effective against the treatment resistant strain, but that it requires a lower IC₅₀ concentration than the non-resistant strain.

The test protocol employed 24-hour incubation with the suspensions prior to virus exposure, adequate time for composition uptake and metabolism by the cells. Uptake and metabolism are thought necessary to invoke antiviral activity of some long-chain alcohols. Minimal increase in cellular proliferation of Vero cells exposed to long-chain alcohols has also been reported.

Thus, the composition according to the present invention demonstrated no cytotoxicity up to, and including, concentrations of 250 mg/ml, the highest concentration tested. The composition according to the present invention was efficacious in reducing plaque formation in HSV-1 infected cells in a concentration-dependant manner, demonstrating specific antiviral activity against both the standard clinical HSV-1 strain (6143) and against the acyclovir resistant strain (15671). The IC₅₀ is the lowest concentration that will either reduce the severity of the virus infection or the number of cells infected by virus particles by 50%. It is also the concentration that results in a 50% reduction in number of plaques caused by the virus. The IC₅₀ of tested composition was 0.51% for strain 6143, and 0.22% for strain 15671.

The discovery that this combination of alcohols either alone or in combination with other compositions, such as salts of long chain fatty acids and/or mixed esters, which are naturally occurring and are essentially non-toxic in concentration ranges of interest, have significant anti-viral effect is considered to be of major import in as much as the way is open to providing a safe and effective method for the treatment for virus diseases and for preventing or at least significantly reducing the likelihood of virus infection to the human or other animal patient, without any significant side effects and without the need for as intense monitoring by the treating physician as is required with inherently toxic compounds.

As a treatment for acquired immunodeficiency syndrome (AIDS), as a method for prophylactic treatment of persons exposed to AIDS and/or carrying AIDS virus but without demonstrating AIDS symptoms, and as methods and compositions for preventing or reducing the risk of infection by AIDS and virus-induced diseases, the present invention is regarded as a significant improvement.

Another important aspect of the invention is that it may provided a safe and effective mode of treatment of diseases resulting from infection of the patient with such lipid-containing virus as HTLV-1, HSV-1, HSV-2, cytomegalovirus (CMV), Epstein-Bar (EBV), and influenza viruses.

The risk of infection by such viruses as HIV, HSV-1, HSV-2, CMV, EBV, influenza viruses and other viruses which are communicated by personal contact, contact with contaminated blood or tissue or laboratory instruments or devices, aerosol transmission, etc., may be substantially reduced by the methods and compositions of the present invention.

## Claims

1. A physiologically compatible medicament composition comprising:
at least one Cₙ monounsaturated alcohol, where 18≤n≤24;
at least one salt of a fatty acid according to the formula R¹-COO⁻M⁺, where:
R¹ comprises CH₃-(CH₂)₇-[CH=CH-CH₂-(CH₂)ₓ]⁻;
x is at least one of 6, 8, 10 and 12;
M⁺ is a monovalent alkali metal ion, and
a physiologically compatible carrier.

2. A physiologically compatible medicament composition according to claim1 wherein said monounsaturated alcohol is of formula CH₃-(CH₂)₇-CH=CH-CH₂₋(CH₂)ₓCH₂OH
wherein x is at least one of 6, 8, 10 and 12.

3. The physiology compatible medicament composition of claim 1 or claim 2 further comprising at least one of a penetration-enhancing compound and wherein the physiologically compatible carrier is injectable, wherein said medicament composition is suitably adapted for at least one of transdermal introduction, intravenous injection, and intramuscular injection into humans or other mammals.

4. The physiologically compatible medicament composition of claim 1 or claim 2, which is a suppository for trans-membranal introduction into at least one of the anus and the vagina of humans or other mammals, wherein said physiologically compatible carrier is substantially solid at ambient room temperature and melts at approximately 37°C.

5. A medicament according to any one of claims 1, 2, 3 and 4 wherein the composition further comprises at least one mixed ester according to the formula R¹-COO-R², where:
R¹ comprises CH₃-(CH₂)₇-[CH=CH-CH₂-(CH₂)ₓ];
x is at least one of 6, 8, 10 and 12; and
R² is at least one of an alkyl group and an aliphatic group.

6. A medicament according to claim 5 wherein said aliphatic group has 1 to 12 carbon atoms.

7. A medicament according to any preceding claim wherein the proportions of said alcohols are 1% octadecenol; 44% eicosenol; 45% docosenol; and 9% tetracosenol.

8. Use of at least one of octadecenol, eicosenol, docosenol, and tetracosenol, and at least one salt of a fatty acid according to the formula R¹⁻COO-M⁺ where:
R¹ comprises CH₃-(CH₂)₇-[CH=CH-CH₂-(CH₂)ₓ]⁻;
x is at least one of 6, 8, 10 and 12; and
M⁺ is a monovalent alkali metal ion,
in the manufacture of an intravenous, intramuscular, trans-mucosal, transdermal or oral composition for preventing, inhibiting or treating virus diseases in humans or animals.

9. Use according to claim 8, involving the addition of at last one mixed ester according to the formula R¹-COO-R² where:
R¹ comprises CH₃-(CH₂)₇-[CH=CH-CH₂-(CH₂)ₓ];
x is at least one of 6, 8, 10 and 12; and
R² is at least one of an alkyl group and an aliphatic group, preferably between 1 and 12 carbon atoms.

10. Use according to claim 8 or claim 9 wherein said alcohols are present in proportions of 1% octadeconol, 44% eicosenol, 45% docosenol, and 9% tetracosenol.

11. Use according to claim 8 for the manufacture of a medicament for preventing or treating viral infections caused by herpes viruses, influenza viruses, human lymphotrophic viruses, human immunodeficiency viruses, papilloma viruses or respiratory syncitial virus.

12. Use according to claim 8 for the manufacture of a medicament for preventing or treating viral infections caused by HSV-1, HSV-2, HSV-6, cytomegelo virus (CMV), Epstein-Barr virus (EBV) or varicella zoster virus (VZV).

## Patentansprüche

1. Physiologisch unbedenkliche Medikamentenzusammensetzung, enthaltend:
wenigstens einen einfach ungesättigten Cₙ-Alkohol, wobei 18≤n≤24;
wenigstens ein Salz einer Fettsäure der Formel R¹-COO⁻M⁺, wobei:
R¹ CH₃-(CH₂)₇-[CH=CH-CH₂-(CH₂)ₓ]⁻ enthält;
x für wenigstens eine der Zahlen 6, 8, 10 und 12 steht;
M⁺ für ein einwertiges Alkalimetallion steht und
einen physiologisch unbedenklichen Träger.

2. Physiologisch unbedenkliche Medikamentenzusammensetzung nach Anspruch 1, wobei der einfach ungesättigte Alkohol die Formel CH₃-(CH₂)₇-CH=CH-CH₂-(CH₂)ₓCH₂OH aufweist,
wobei x für wenigstens eine der Zahlen 6, 8, 10 und 12 steht;

3. Physiologisch unbedenkliche Medikamentenzusammensetzung nach Anspruch 1 oder 2, weiterhin enthaltend wenigstens eine die Penetration verbessernde Verbindung, wobei der physiologisch unbedenkliche Träger injizierbar ist, wobei die Medikamentenzusammensetzung geeigneterweise für die transdermale Verabreichung, intravenöse Injektion und/oder intramuskuläre Injektion in Menschen oder andere Säugetiere ausgelegt ist.

4. Physiologisch unbedenkliche Medikamentenzusammensetzung nach Anspruch 1 oder 2, bei der es sich um ein Zäpfchen für die transmembrane Verabreichung in den Anus und/oder die Vagina von Menschen oder anderen Säugetieren handelt, wobei der physiologisch unbedenkliche Träger bei Raumtemperatur im wesentlichen fest ist und bei ungefähr 37°C schmilzt.

5. Medikament nach einem der Ansprüche 1, 2, 3 und 4, wobei die Zusammensetzung weiterhin wenigstens einen gemischten Ester der Formel R¹-COO-R² enthält, wobei:
R¹ für CH₃-(CH₂)₇-[CH=CH-CH₂-(CH₂)ₓ] steht;
x für wenigstens eine der Zahlen 6, 8, 10 und 12 steht und
R² für eine Alkylgruppe und/oder eine aliphatische Gruppe steht.

6. Medikament nach Anspruch 5, wobei die aliphatische Gruppe 1 bis 12 Kohlenstoffatome aufweist.

7. Medikament nach einem der vorhergehenden Ansprüche, wobei die Verhältnisse der Alkohole 1% Octadecenol; 44% Eicosenol; 45% Docosenol; und 9% Tetracosenol betragen.

8. Verwendung von wenigstens einem Octadecenol, Eicosenol, Docosenol und Tetracosenol und wenigstens einem Salz einer Fettsäure der Formel R¹-COO⁻M⁺, wobei:
R¹ für CH₃-(CH₂)₇-[CH=CH-CH₂-(CH₂)ₓ]⁻ steht;
x für wenigstens eine der Zahlen 6, 8, 10 und 12 steht; und
M⁺ für ein einwertiges Alkalimetallion steht,
bei der Herstellung einer intravenösen, intramuskulären, transmukosalen, transdermalen oder oralen Zusammensetzung zur Prävention, Inhibierung oder Behandlung von Viruskrankheiten in Menschen oder Tieren.

9. Verwendung nach Anspruch 8, bei der man wenigstens einen gemischten Ester der Formel R¹-COO-R² zusetzt, wobei:
R¹ für CH₃-(CH₂)₇-[CH=CH-CH₂-(CH₂)ₓ] steht;
x für wenigstens eine der Zahlen 6, 8, 10 und 12 steht; und
R² für eine Alkylgruppe und/oder eine aliphatische Gruppe steht, die vorzugsweise 1 bis 12 Kohlenstoffatome aufweist.

10. Verwendung nach Anspruch 8 oder 9, wobei die Alkohole in Verhältnissen von 1% Octadecenol; 44% Eicosenol; 45% Docosenol; und 9% Tetracosenol vorliegen.

11. Verwendung nach Anspruch 8 zur Herstellung eines Medikaments zur Prävention oder Behandlung von Virusinfektionen, die durch Herpesviren, Grippeviren, humane lymphotrophe Viren, Human-Immunodeficiency-Viren, Papillomaviren oder Respiratory-Syncitial-Virus verursacht werden.

12. Verwendung nach Anspruch 8 zur Herstellung eines Medikaments zur Prävention oder Behandlung von Vireninfektionen, die durch HSV-1, HSV-2, HSV-6, Cytomegalovirus (CMV), Epstein-Barr-Virus (EBV) oder Varicella-Zoster-Virus (VZV) verursacht werden.

## Revendications

1. Composition médicamenteuse physiologiquement compatible, comprenant :
au moins un alcool monoinsaturé en Cₙ, où 18≤n≤24 ;
au moins un sel d'un acide gras selon la formule R¹-COO⁻M⁺, dans laquelle :
R¹ comprend CH₃-(CH₂)₇-[CH=CH-CH₂-(CH₂)ₓ]⁻;
x est au moins l'un parmi 6, 8, 10 et 12 ;
M⁺ est un ion de métal alcalin monovalent ; et
un véhicule physiologiquement compatible.

2. Composition médicamenteuse physiologiquement compatible selon la revendication 1, **caractérisée en ce que** ledit alcool monoinsaturé répond à la formule CH₃-(CH₂)₇-CH=CH-CH₂-(CH₂)ₓCH₂OH
dans laquelle x est au moins l'un parmi 6, 8, 10 et 12.

3. Composition médicamenteuse physiologiquement compatible selon la revendication 1 ou la revendication 2, comprenant en outre au moins un parmi un composé améliorateur de pénétration et dans laquelle le véhicule physiologiquement compatible est injectable, **caractérisée en ce que** ladite composition médicamenteuse est convenablement adaptée pour au moins une parmi une introduction transdermique, une injection intraveineuse, et une injection intramusculaire chez l'être humain ou d'autres mammifères.

4. Composition médicamenteuse physiologiquement compatible selon la revendication 1 ou la revendication 2, qui est un suppositoire destiné à une introduction transmembranaire dans au moins l'un parmi l'anus et le vagin de l'être humain ou d'autres mammifères, **caractérisée en ce que** ledit véhicule physiologiquement compatible est sensiblement solide à température ambiante et fond à environ 37°C.

5. Médicament selon l'une quelconque des revendications 1, 2, 3 et 4, **caractérisé en ce que** la composition comprend en outre au moins un ester mixte selon la formule R¹-COO-R², dans laquelle :
R¹ comprend CH₃-(CH₂)₇-[CH=CH-CH₂-(CH₂)ₓ] ;
x est au moins l'un parmi 6, 8, 10 et 12 ; et
R² est au moins l'un parmi un groupement alkyle et un groupement aliphatique.

6. Médicament selon la revendication 5, **caractérisé en ce que** ledit groupement aliphatique a de 1 à 12 atomes de carbone.

7. Médicament selon l'une quelconque des revendications précédentes, **caractérisé en ce que** les proportions desdits alcools sont 1% d'octadécénol ; 44% d'éicosénol ; 45% de docosénol ; et 9% de tétracosénol.

8. Utilisation d'au moins un parmi l'octadécénol, l'éicosénol, le docosénol et le tétracosénol, et d'au moins un sel d'un acide gras selon la formule R¹-COO⁻M⁺, dans laquelle :
R¹ comprend CH₃-(CH₂)₇-[CH=CH-CH₂-(CH₂)ₓ]⁻ ;
x est au moins l'un parmi 6, 8, 10 et 12 ; et
M⁺ est un ion de métal alcalin monovalent ;
dans la fabrication d'une composition intraveineuse, intramusculaire, trans-muqueuse, transdermique ou orale destinée à la prévention, l'inhibition ou le traitement de maladies virales chez l'être humain ou l'animal.

9. Utilisation selon la revendication 8, impliquant l'addition d'au moins un ester mixte selon la formule R¹-COO-R², dans laquelle :
R¹ comprend CH₃-(CH₂)₇-[CH=CH-CH₂-(CH₂)ₓ] ;
x est au moins l'un parmi 6, 8, 10 et 12 ; et
R² est au moins l'un parmi un groupement alkyle et un groupement aliphatique, comprenant préférablement entre 1 et 12 atomes de carbone.

10. Utilisation selon la revendication 8 ou la revendication 9, **caractérisée en ce que** lesdits alcools sont présents selon des proportions de 1% d'octadécénol, 44% d'éicosénol, 45% de docosénol, et 9% de tétracosénol.

11. Utilisation selon la revendication 8, pour la fabrication d'un médicament destiné à la prévention ou au traitement d'infections virales provoquées par les virus de l'herpès, les virus grippaux, les virus lymphotrophiques humains, les virus d'immunodéficience humaine, les virus de papillome ou le virus respiratoire syncytial.

12. Utilisation selon la revendication 8, pour la fabrication d'un médicament destiné à la prévention ou au traitement d'infections virales provoquées par HSV-1, HSV-2, HSV-6, le cytomégalovirus (CMV), le virus d'Epstein-Barr (EBV) ou le virus de varicelle-zona (VZV).
